Europäisches Patentamt

European Patent Office

Office européen des brevets

(19)

(11) Publication number: **0 026 547**
**B1**

(12) **EUROPEAN PATENT SPECIFICATION**

(45) Date of publication of patent specification: **01.02.84**

(21) Application number: **80200911.8**

(22) Date of filing: **26.09.80**

(51) Int. Cl.³: **C 07 F 3/00, C 07 C 31/30,
C 07 C 29/68 //C08G65/28,
C07C41/03, B01J31/02**

(54) **Process for the preparation of basic salts of alkaline earth metals.**

(30) Priority: **27.09.79 US 79497**

(43) Date of publication of application:
**08.04.81 Bulletin 81/14**

(45) Publication of the grant of the patent:
**01.02.84 Bulletin 84/5**

(84) Designated Contracting States:
**BE DE FR GB IT NL SE**

(56) References cited:
**EP - A - 0 001 861
FR - A - 879 689
FR - A - 1 176 300
FR - A - 1 225 135
FR - A - 1 366 307
FR - A - 1 555 094
GB - A - 767 601
GB - A - 1 501 327
US - A - 3 009 887
US - A - 3 100 750
US - A - 3 803 250
US - A - 4 020 115
US - A - 4 126 627
US - A - 4 223 164**

(73) Proprietor: **UNION CARBIDE CORPORATION
Old Ridgebury Road
Danbury Connecticut 06817 (US)**

(72) Inventor: **McCain, James Herndon
1987 Parkwood Road
Charleston West Virginia 25314 (US)**
Inventor: **Foster, Donald Joseph
603 39th Street, S.E.
Charleston West Virginia 25304 (US)**

(74) Representative: **Urbanus, Henricus Maria, Ir. et al,
c/o Vereenigde Octrooibureaux Nieuwe Parklaan
107
NL-2587 BP 's-Gravenhage (NL)**

(56) References cited:
**CHEMICAL ABSTRACTS, vol. 74, no. 24, 14th
June 1971, page 9, no. 126177j Columbus,
Ohio, U.S.A. K.S. KAZANSKII et al.:
"Polymerization of ethylene oxide in the
presence of alkaline earth alcoholates"
HOUBEN WEYL, Methoden der Oragnischen
Chemie, Band VI/2, Sauerstoff-Verbindungen 1,
Teil 2, 1963, pages 13-16 Stuttgart, DE
Encyclopedia of Chemical Technology, Kirk-
Othmer; 3rd Edition, Volume 2, pages 8 and 9**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Process for the preparation of basic salts of alkaline earth metals

Background of the invention

This invention relates to the preparation of compositions which are catalytically active for the preparation of condensation reaction products of an epoxide and organic compounds having an active hydrogen with a restricted molecular weight distribution and reduced by-products.

A variety of products such as surfactants, functional fluids, glycol ethers, polyols, and the like are commercially prepared by the condensation reaction of epoxides with organic compounds having an active hydrogen, generally in the presence of an alkaline or acidic catalyst. The types of products prepared and properties thereof depend on the active hydrogen compound, the epoxide, and the number of moles of epoxide employed as well as the catalyst, a mixture of condensation products species being obtained containing different molecular proportions of epoxide. Thus, the reaction products generally obtained have a wide range of molecular weights and of molecular distribution of the epoxide units.

It is generally desirable to restrict the molecular distribution of the mixture to adjacent analogues of the desired product, insofar as possible, but this is quite difficult to control. Acidic catalysts tend to give a narrower molecular distribution than alkaline catalysts, but also contribute to the formation of undesired by-products. Thus, alkaline catalysts are generally used as the more efficient type of catalyst, but the molecular distribution in the resulting products are more diffuse.

Heretofore, several methods have been suggested for providing reaction products of an active hydrogen compound and epoxides having a narrower range of molecular weights and molecular distribution of the epoxide units, or which reduce or eliminate the production of undesirable poly(ethylene) glycol and cyclic and straight chain ether by-products. For example, in U.S. Patent 4,112,231 to Weibull et al it is disclosed that the use of certain neutral inorganic fluoborate and perchlorate salts will catalyze the reaction of epoxides with active hydrogen compounds to give products having a narrower molecular distribution and a larger proportion of desired species; in U.S. Patent No. 3,682,849 to Smith et al improved ethoxylated derivatives of $C_{11}$—$C_{18}$ alcohols are prepared by removing unreacted alcohol and lower ethoxylates from the conventionally produced ethoxylate mixture using vapor phase separation techniques; in U.S. Patent 2,870,220 to Carter, a two-stage process is disclosed for preparing monoalkyl ethers of ethylene glycol and polyethylene glycols of more restricted molecular weight range wherein an alkanol and ethylene oxide are reacted in the presence of an acidic catalyst during the first stage and then in the second-stage, after removal of acid catalyst and unreacted alkanol, reacting the mixture with ethylene oxide in the presence of an alkali metal alcoholate of the initial alkanol; and in U.K. Patent 1,501,327 to Laemmle et al is disclosed a method of preparing mono- and poly-glycol ethers substantially free of undesired alkylene glycols by-products which involves heating a reaction mixture containing an alkylene oxide and an alcohol in the presence of a catalyst containing alkali or alkaline earth cations wherein some or all of the catalyst is an anhydrous high boiling liquid residue prepared by concentrating the liquid residue left from the same or different etherification process after removal of the glycol ether product from the reaction mixture.

There has also been suggested basic compounds of alkali and alkaline earth metals that have been used, for example, in the polymerization of alkylene oxides (see U.S. Patent 3,100,750 to Bailey, Jr. et al and French Patent 1,176,300) and the preparation of nonionic surfactants having special properties (see U.S. Patent 4,134,854 to Milligan). To the best of our knowledge, however, none of the processes or special catalysts disclosed in the art are completely satisfactory in that they require multistage procedures or special acid-resistant equipment, give undesirable by-products or simply do not provide sufficient control over the molecular weight distribution. It would be highly desirable, therefore, to develop a process for the preparation of compositions which are catalytically active in the reaction of an epoxide with an organic compound having an active hydrogen wherein such reaction could be more readily carried out to prepare products that have a narrow molecular weight distribution of analogue species and contain only small amounts, at most, of undesirable by-products.

Summary of the invention

The present invention is directed to the novel method for the preparation of a class of catalysts which are extremely effective for the preparation of oxyalkylation reaction products having a narrower molecular weight distribution and reduced amount of undesirable by-products.

In accordance with the present invention there is provided a process for preparing catalytically active compounds of alkaline earth metals in which metallic calcium, strontium and/or barium is reacted with a lower alkanol having 1 to 7 carbon atoms, to form a metal alkoxide, characterized in that to the reaction product obtained there is added a polyol or a higher aliphatic, cycloaliphatic or phenyl-substituted aliphatic monohydric alcohol having at least 4 carbon atoms and which is less volatile than the lower alkanol and that the alkanol is removed from the mixture.

It has been discovered that the basic salts of alkaline earth metals herein described not only catalyze the reaction of a monohydric alcohol, polyol, or alkylphenol and an epoxide but also favor a narrower molecular distribution, i.e., a more limited range of molecular species and a larger proportion of the desired species in the reaction product. Moreover, the oxyalkylation reaction can be carried out in a single stage without the need for special acid-resistant equipment and the products produced thereby have been found, in general, to contain only small amounts of undesired poly(alkylene)glycol and ether by-products.

In the process of the invention metallic calcium, strontium, barium or their hydrides or acetylides are reacted with a lower alkanol having 1 to 7 carbon atoms to form the alkaline earth metal alkoxide; mixing a polyol or a higher aliphatic, cycloaliphatic or phenyl-substituted aliphatic monohydric alcohol having at least 4 carbon atoms and which is less volatile than the lower alkanol with the lower alkanol alkaline earth metal alkoxide, and removing the lower alkanol from the reaction mixture.

The lower alkanols which can be used are primary, secondary or tertiary alkanols having 1 to 7 carbon atoms and preferably 1 to 4 carbon atoms which are branched or straight chain. Exemplary of preferred alkanols are methanol, ethanol, n-propanol, isopropanol, n-butanol, and iso-butanol. Also suitable are n-pentanol, 2-pentanol, 3-pentanol and n-hexanol.

Suitable higher monohydric alcohols having at least 4 carbon atoms that may be used in accordance with the invention can be primary and secondary aliphatic alcohols which are straight or branched chain having at least 4 and preferably from 8 to 30 carbon atoms. Exemplary of such primary straight chain alcohols are n-octanol, nonanol, decanol, undecanol, dodecanol, tridecanol, tetradecanol, pentadecanol, hexadecanol, and octadecanol; and of such branched chain or secondary alcohols are 2-ethylhexanol, isooctanol, sec-octanol, and isodecanol. Particularly suitable are linear and branched primary alcohols and alcohol mixtures such as produced by the "Oxo" reaction of normal $C_3$—$C_{20}$ olefins.

Also suitable are less volatile cycloaliphatic monohydric alcohols, including, for example, cycloheptanol and cyclooctanol, as well as less volatile phenyl-substituted monohydric alcohols such as benzyl alcohol, phenylethyl alcohol, and phenpropyl alcohol.

Polyols suitable for use in accordance with the present invention can have from two to thirty carbon atoms and from two to six hydroxyl groups including, for example, glycerine, glycols such as ethylene glycol, propylene glycol, butylene glycol, pentylene glycol, hexylene glycol, heptylene glycol, neopentylene glycol, decylene glycol, diethylene glycol, triethylene glycol, dipropylene glycol, tripropylene glycol, pentaerythritol, galactitol, sorbitol, mannitol, erythritol, trimethylolethane and trimethylolpropane.

The process of the invention involves, in general, two steps. In the first step, calcium, strontium, or barium containing raw materials as hereinabove described are reacted with a lower alkanol having 1 to 7 and preferably to about 4 carbon atoms for the time necessary to form the alkaline earth metal alkoxide. The temperature and pressure at which the reaction between the metal and the lower alkanol is conducted is not critical and can vary widely. In general, the higher the temperature the faster the rate of reaction. It is usually convenient and preferred to carry out the reaction in a temperature-pressure region where the alkanol is a liquid.

The amount of alkaline earth metal to be reacted with the lower alkanol is not narrowly critical but can vary widely. However, since the lower alkanol is to be removed during a further step of the catalyst preparation, it is preferred to use a larger amount of metal consistent with having a mixture with reasonable handling characteristics. In general, the concentration of metal in the lower alkanol may vary from 0.01 to 20 percent by weight, and preferably from 0.1 to 10 percent by weight of alkanol.

In the next step of the process, the metal alkoxide in the lower alkanol is mixed with a polyol or a higher aliphatic, cycloaliphatic or phenyl-substituted aliphatic monohydric alcohol having at least 4 carbon atoms which is less volatile than the lower alkanol employed the first step of the process and the lower alkanol introduced with the lower alkanol metal alkoxide is removed from the mixture. Removal of the lower alkanol may be conducted by any separation means that retains the catalytic activity of the alkaline earth metal alkoxide. Preferably, distillation techniques are used, but other methods such as column chromatography and fractional freezing may also be employed. The lower alkanol is removed from the mixture at a temperature and pressure whereby, for example, the lower alkanol is vaporized and the polyol or the higher alcohol is not, the temperature being dependent on the pressure and types of polyols or higher alcohols and lower alkanols that were used in the reactions.

While a variety of higher aliphatic, cycloaliphatic or phenyl-substituted aliphatic monohydric alcohols or polyols as herein described may be employed in preparing the soluble metal alkoxide, it is usually preferred to employ the polyols, alcohols or mixtures thereof that are intended to be employed in the reaction with an epoxide to be catalyzed. The amount of higher aliphatic, cycloaliphatic or phenyl-substituted aliphatic monohydric alcohol or polyol mixed with the lower $C_{1-7}$ alkanol metal alkoxide reaction mixture should be sufficient to convert all of the $C_{1-7}$ alkanol metal alkoxide to the metal alkoxide of the less volatile polyol or higher alcohol and preferably at least 10 percent greater than that stoichiometrically required.

Alternatively, the higher alcohol or polyol mixed with the metal alkoxide in $C_{1-7}$ alkanol reaction product is the monohydric alcohol or polyol to be oxyalkylated which is added in the amount sufficient to give the desired level of catalyst in the monohydric alcohol or polyol, after removal of the $C_{1-7}$ alkanol, for the oxyalkylation reaction. The desired concentration of catalyst in the oxyalkylation reaction mixture is not critically narrow, and can, in general, vary from 0.001 percent to 10 percent by weight of calcium, strontium, or barium based on the reactive hydrogen compound.

The solution of calcium, strontium, or barium alkoxide in the higher aliphatic, cycloaliphatic or phenyl-substituted aliphatic monohydric alcohol or polyol so prepared is a basic alkaline salt of the alkaline earth metal which is soluble in the reactants employed during the reaction of an alkylene oxide and a reactive hydrogen compound such as monohydric alcohol and in the reaction products prepared thereby.

The soluble basic salt of alkaline earth catalyst prepared in accordance with the practice of the invention catalyzes the reaction of an organic compound having an active compound selected from the group consisting of monohydric alcohols, polyols, and phenols with an epoxide. The reaction may be conducted in a conventional manner, that is, the active hydrogen compound and the catalyst are placed in a reactor, alkylene oxide is added at the reaction temperature until the desired number of moles have been added, and the product is removed from the reactor and neutralized. The reaction may be conducted in the presence of a solvent, but usually a solvent is not employed.

The temperature at which the reaction proceeds is not narrowly critical and generally products can be made at a reasonable rate of reaction and without decomposition of the product at a temperature between 50°C and 270°C with a temperature between 100°C and 200°C being generally preferred. While the pressure of the reaction is not narrowly critical, when low-boiling epoxides such as ethylene oxide and propylene oxide are employed, a pressurized reactor is preferably used.

The product made may be neutralized with any acid that will convert the catalyst to a neutral salt, as for example, acetic acid, carbon dioxide, sulfuric acid, phosphoric acid and phenol.

The novel catalysts of the invention are useful in effecting the oxyalkylation of primary and secondary monohydric aliphatic alcohols which are straight or branched chain and having from one to thirty carbon atoms. Exemplary of such primary straight chain monohydric alcohols are methanol, ethanol, butanol, pentanol hexanol, heptanol, octanol, nonanol, decanol, undecanol, dodecanol, tridecanol, tetradecanol, pentadecanol, hexadecanol, and octadecanol; and of such branched chain or secondary alcohols are isopropyl alcohol, 2-ethylhexanol sec-butanol, iso-butanol, 2-pentanol, 3-pentanol, iso-octanol, sec-octanol, and isodecanol. Particularly suitable are linear and branched primary alcohols and alcohol mixtures such as are produced by the "Oxo" reaction of normal $C_3$—$C_{20}$ olefins.

The process of the invention is also applicable to cycloaliphatic monohydric alcohols, including for example, cyclohexanol, cyclopentanol, cycloheptanol, cyclopropanol and cyclooctanol, as well as phenyl-substituted nonohydric alcohols such as benzyl alcohol, phenylethyl alcohol, and phenylpropyl alcohol.

Applicable phenols include, for example phenol, alkyl phenols such as p-methylphenol p-ethylphenol, p-butylphenol, p-heptylphenol, p-nonylphenol, dinonylphenol and p-decylphenol. The aromatic radicals may contain other conventional substituents such as halide atoms.

The polyols to which the present invention is applicable can have from two to thirty carbon atoms and from two to six hydroxyl groups including, for example, glycerine, glycols such as ethylene glycol, propylene glycol, butylene glycol, pentylene glycol, hexylene glycol, heptylene glycol, neopentylene glycol, decylene glycol, diethylene glycol, triethylene glycol, dipropylene glycol, tripropylene glycol, pentaerythritol, galactitol, sorbitol, mannitol, erythritol, trimethylolethane and trimethylolpropane.

The epoxides which are applicable in accordance with the invention can be any epoxide having from two to about thirty carbon atoms. Exemplary epoxides include alkylene oxides such as ethylene oxide; propylene oxide-1,2; butylene oxide-1,2 and -2,3 pentylene oxide-1,2; hexylene oxide-1,2; octylene oxide-1,2; and decylene oxide -1,2; and mixtures of the same; epoxidized fatty alcohols derived from fatty oils such as epoxidized soybean fatty alcohols and epoxidized linseed oil fatty alcohols; cycloalkylene epoxides including, for example, cyclohexane oxide, cyclopentene oxide, cycloheptene oxide; aromatic epoxides such as styrene oxide and 2-methyl styrene oxide; and hydroxy- and halogen-substituted epoxides such as glycidol, epichlorohydrin and epibromhydrin.

The amount of catalyst used in accordance with the invention is not narrowly critical and a catalytic effect has been noted with only a small amount thereof being present. In general, the catalyst concentration can vary from 0.001 percent to 10 percent by weight of calcium, strontium, and barium based on the weight of active hydrogen compound. Concentrations of alkaline earth metal within the range from 0.05 percent to 5.0 percent by weight of active hydrogen compound are usually preferred. The reaction rate, however, is dependent on both temperature and catalyst concentration and to achieve a given rate, more catalyst is required at a low temperature than at a high temperature.

The invention will become more clear when considered together with the following examples which are set forth as being merely illustrative of the invention and which are not intended, in any manner, to be limitative thereof. Unless otherwise indicated, all parts and percentages are by weight.

Example 1

To 500 cc of ethanol heated at 74° to 78°C in a 1-liter reaction flask equipped with a stirrer, thermometer, nitrogen inlet, and condenser was added 20 grams (0.5 moles) of calcium metal over a 1.5 hour period. The calcium dissolved in the ethanol with evolution of hydrogen to give a cloudy solution of calcium ethoxide, which on cooling precipitated. The solid calcium ethoxide was collected by filtration and dried in a vacuum oven at 25°C.

To 1050 grams (4.95 moles) of a mixture of $C_{12}$ to $C_{15}$ primary alcohols (60% branched, 40% normal isomers) available under the tradename LIAL-125 from Liquichemica Italia in a 2-liter, stirred reactor, equipped with a thermometer, a nitrogen inlet and a dry ice trap connected to a vacuum source, was added 25 grams (0.19 moles) of the calcium ethoxide prepared above. The ethanol introduced with the calcium ethoxide was removed by heating the mixture at 90°C and 3 mm pressure for two-thirds of an hour. The resulting sample was a substantially homogeneous solution although it contained some haze.

The solution of calcium in alcohol prepared above was transferred to a steel, 9.5 liter autoclave equipped with a stirrer, an automatic temperature controller, and an automatic feed controller. The autoclave was heated to 110°C, pressurized to 137 kPa with nitrogen and then to 413 kPa with ethylene oxide. By automatic control, 1685 grams (38.3 moles) of ethylene oxide was fed to the autoclave at 110°C over a period of 2.5 hours. After cookout to 234 kPa, the product (2717 grams) was cooled, drained from the reactor, and neutralized to a pH of 7 with acetic acid in a stirred flask. The calcium catalyst remained dissolved in the reaction mixture until the neutralization step.

In a second run, 8.2 grams (0.15 moles) of potassium hydroxide in 1000 grams of the mixture of $C_{12}$ to $C_{15}$ primary alcohols hereinabove described was charged into the 9.5 liter autoclave and heated to 110°C under 413 kPa pressure as above. 1685 grams (38.3 moles) of ethylene oxide was then fed to the autoclave and reacted with the alcohol at 110°C over a period of 0.65 hours. After cookout, the product (2707 grams) was cooled, drained from the reactor and neutralized to a pH of 7 with acetic acid.

Gel permeation chromatography, a standard technique for determining the molecular weight distribution of polymers and surfactants was used to evaluate the reaction products. A comparison of the widths of gel permeation peaks at their half-heights, all run at constant conditions, is a measure of the relative broadness of the molecular weight distribution of the polymers or surfactants. When the performance of the instrument is calibrated with standards of known molecular weights, it is possible to define the molecular weight range represented by the peak width at half height.

Gel permeation chromatography results on the products of run #1 and #2 hereof are reported in Table I, below.

TABLE I
Gel permeation chromatography results

| Catalyst | Peak width at one-half height (cc) | Molecular weight range at one-half height |
|---|---|---|
| Basic calcium alkoxide (run #1) | 2.9 | 360—820 |
| Potassium hydroxide (run #2) | 4.4 | 320—1000 |

The catalytic effect using the basic calcium alkoxide and potassium hydroxide is apparent from the above results. Although the catalytic activity of the basic calcium alkoxide is less than that of potassium hydroxide, the more favorable molecular distribution of the reaction products obtained with this catalyst as compared to potassium hydroxide is apparent.

Example 2

To a 2-liter glass reaction flask equipped with a condenser, drying tube and nitrogen inlet was added 1000 ml of ethanol and 11 grams (0.08 moles) of barium metal granules. The barium and ethanol started reacting immediately at ambient temperature and were allowed to continue until all the metal was reacted. A clear solution was formed to which was added 550 grams (2.96 moles) of 1-decanol. The condenser was replaced on the reactor flask with a distillation head, and ethanol was distilled from the flask. To completely remove the ethanol, the flask contents were held at 110°C at 2 mm vacuum for 0.25 hours. A clear solution of barium alkoxide in dodecanol was thus formed.

The solution of barium alkoxide contained 0.138 moles of barium per 1000 grams of 1-dodecanol as determined by titration with 0.10 N aqueous hydrochloric acid and a phenolphthaleine indicator. A 500 gram (2.69 moles of dodecanol) portion of the solution was placed in the 9.5 liter autoclave of Example 1 and pressurized as described in Example 1. A total of 770 grams (17.5 moles) of ethylene oxide was added over a period of 0.72 hours while heating the autoclave at 140°C. After a period of heating at 140°C to completely react the ethylene oxide, the reactor was cooled and a 506 gram sample of reaction product was removed, neutralized to a pH of 77 with phosphoric acid and filtered. Results of an analysis of the product are reported in Table II, below.

To the product remaining in the autoclave (764 grams) was added 767 grams (13.2 moles) of propylene oxide at 140°C and 413 kPa pressure over a period of 1.4 hours. After cookout, the product was removed from the autoclave, neutralized to a pH of 7 with phosphoric acid and filtered. Results of an analysis of the product are reported in Tables II and III.

5

In a second test run, the procedure of test run #1 above was followed using 7.0 grams (0.08 moles) of strontium metal granules instead of barium metal. The clear dodecanol solution of strontium alkoxide formed thereby, which contained 0.135 moles of strontium per 1000 grams of dodecanol, was reacted with 762 grams (17.3 moles) of ethylene oxide and then with 784 grams (13.5 moles) of propylene oxide using the procedure described in test run #1 hereinabove. Results of the analysis of the products produced thereby are reported in Tables II and III.

In a third test run, 15 grams of barium hydroxide were mixed with 550 grams of 1-dodecanol in the 2-liter reaction flask described above. After heating at 110°C for 2 hours, the barium hydroxide did not substantially dissolve in the dodecanol. A portion of the mixture of dodecanol and barium hydroxide which contained 2.2 weight percent of barium was reacted with ethylene oxide using the procedure described in test run #1 above and results of an analysis of the products formed are reported in Tables II and III, below.

In a fourth test run, 3.2 grams of calcium metal was reacted with 1000 ml of methanol at reflux to prepare calcium methoxide and then 550 grams of 1-dodecanol were added thereto and the methanol was removed by distillation to a final temperature of 110°C at 2 mm vacuum. A dispersion in dodecanol was prepared which was then reacted with ethylene oxide using the procedure of test run #1 hereof. The further reaction with propylene oxide was not performed. It was found that the dodecanol dispersion with calcium prepared as described above in this test run did not react with ethylene oxide at 140°C but that at 170°C, reaction with ethylene oxide did take place. Results of an analysis of the products produced in this test run are reported in Tables II and III.

In a fifth test run, generally using the procedure of test run #1, hereof 0.072 moles of calcium metal granules were reacted with 140 ml of ethanol at reflux to form a clear solution. 500 Grams of 1-dodecanol were then added to the calcium ethoxide solution and the ethanol was removed by distillation. The resulting solution of calcium alkoxide in dodecanol was charged to the 9.5 liter autoclave of this example and 770 grams of ethylene oxide were added at 140°C and 413 kPa. After completion of the reaction of the ethylene oxide the reaction mixture was cooled, discharged from the autoclave and neutralized with acetic acid. Results of an analysis of the product are reported in Tables II and III.

In a sixth test run, 0.072 moles of potassium hydroxide were added to 500 grams of 1-dodecanol and the mixture was stripped in a stirred flask at 110°C and high vacuum. The mixture was charged to the autoclave of this Example and 757 grams of ethylene oxide were added at 140°C and 413 kPa. An analysis of products produced thereby are reported in Tables II and III.

TABLE II

| Catalyst | Concentration weight percent | Reaction rate [a] | Molecular weight | Moles of ethylene oxide | Appearance | Pour point °C (°F) [b] | Cloud point °C [c] | Moles of propylene oxide | Molecular weight |
|---|---|---|---|---|---|---|---|---|---|
| barium ethoxide | 1.5 | 360 | 488 | 6.9 | slight haze | 10.6 (51) | 54 | 6.7 | 892 |
| strontium ethoxide | 0.97 | 130 | 482 | 6.7 | clear | 10.6 (51) | 52 | 6.7 | 902 |
| barium hydroxide | 2.2 | 83 | 480 | 6.7 | clear | — | 50 | — | — |
| a'] calcium methoxide | 0.48 | no reaction at 140°C | | — | — | — | — | — | — |
| b'] calcium methoxide | 0.48 | 68 [e] | 472 | 6.5 | haze | — | 57 | — | — |
| calcium ethoxide | 0.48 | 82 | 481 | 6.7 | clear | 12.8 (55) | 53 | — | — |
| potassium hydroxide | 0.47 | 330 | 480 | 6.7 | slight haze | 15 (59) | 52 | — | — |

[a] moles of ethylene oxide per mole of catalyst per hour
[b] ASTM D-9766
[c] 1% solution ASTM D-2024
[e] at 170°C.

# 0 026 547

TABLE III

Gel permeation chromatography results

| Catalyst | Concentration (weight percent) | Peak width at one-half height (cc) | Molecular weight range at one-half height |
|---|---|---|---|
| Barium ethoxide | 1.51 | 3.3 | 280—690 |
| Strontium ethoxide | 0.97 | 3.1 | 290—690 |
| Barium hydroxide | 2.2 | 2.9 | 290—640 |
| b) Calcium methoxide | 0.48 | 3.3 | 280—690 |
| Calcium ethoxide | 0.48 | 2.9 | 290—640 |
| Potassium hydroxide | 0.47 | 4.5 | 270—930 |

The results show that basic salts of barium, strontium, and calcium catalyze the oxyethylation of the monohydric alcohol to give reaction products having a narrower molecular weight distribution and lower pour points compared to products made with potassium hydroxide. It is also shown, however, that barium hydroxide and calcium methoxide, which do not form a soluble salt with dodecanol, are not as active catalysts as the soluble salts of barium and calcium.

Example 3

A mixture of 1.5 liters of calcium ethoxide in ethanol (prepared by reacting 28 grams (0.70 moles) of calcium metal with 1.5 liters of ethanol at reflux) and 600 grams (6.5 moles) of glycerine is heated under vacuum to remove the ethanol.

Five hundred and sixty five grams (6.14 moles of glycerine) of the solution containing glycerine and basic salt of calcium is charged into a 7.5 liter steel autoclave reactor equipped with an automatic temperature controller, an automatic pressure and reactant feed controller, and circulating means for the reactants. The autoclave is heated to 117°C, pressurized to 845 kPa with nitrogen and then to 482 kPa with propylene oxide. Over a 2 hour period, 365 grams (6.3 moles) of propylene oxide is fed to the autoclave while maintaining the temperature at 117°C. The temperature of the autoclave is then raised to 135°C and an additional 3390 grams (58.4 moles) of propylene oxide is added over a period of 5 hours while maintaining the temperature at 135°C and a constant pressure. After all the propylene oxide is added, the autoclave is held at 135°C for one additional hour to achieve complete reaction after which the reactor is cooled and the reaction mixture is discharged and analyzed.

The reaction product is a clear solution which is determined to have a hydroxyl number of 238 from which a molecular weight of 707 is calculated assuming a functionality of 3.

Example 4

To a 2-liter glass flask equipped with a distillation head was charged 1100 grams (5.2 moles) of the mixture of $C_{12}$ to $C_{15}$, primary alcohol of Example 1 and 20.8 grams (0.52 moles) of calcium metal granules. The contents of the flask were heated to 120°C and no reaction occurred as evidenced by zero gas flow at constant temperature. Over a period of 0.5 hours, the temperature was raised to 190°C but there was still no evidence of a reaction.

## Claims

1. Process for preparing catalytically active compounds of alkaline earth metals in which metallic calcium, strontium, barium or their hydrides or acetylides are reacted with a lower alkanol having 1 to 7 carbon atoms, to from a metal alkoxide, characterized in that to the reaction product obtained there is added a polyol or a higher aliphatic, cycloaliphatic or phenyl-substituted aliphatic monohydric alcohol having at least 4 carbon atoms and which is less volatile than the lower alkanol and that the alkanol is removed from the mixture.

2. The process of claim 1 in which the amount of polyol or less volatile aliphatic, cycloaliphatic or phenyl-substituted aliphatic monohydric alcohol added to the $C_{1-7}$ alkanol-alkaline earth metal alkoxide reaction product is an amount sufficient to convert all of the $C_{1-7}$ alkanol metal alkoxide.

3. The process of claim 1 in which the concentration of alkaline earth metal in the $C_{1-7}$ alkanol reaction mixture is from 0.01 to 20 percent by weight.

4. The process of claim 1 in which a polyol is mixed with the $C_{1-7}$ alkanol-alkaline earth metal alkoxide reaction mixture.

5. The process of claim 4 in which the polyol has from 2 to 30 carbon atoms and from 2 to 6 hydroxyl groups.

6. The process of claim 1 in which a less volatile aliphatic, cycloaliphatic or phenyl-substituted aliphatic monohydric alcohol is mixed with the $C_{1-7}$ alkanol-alkaline earth metal alkoxide reaction mixture.

7. The process of claim 6 in which the less volatile aliphatic, cycloaliphatic or phenyl-substituted aliphatic monohydric alcohol has between 8 and 30 carbon atoms.

8

**0 026 547**

**Patentansprüche**

1. Verfahren zur Herstellung von katalytisch aktiven Verbindungen von Erdalkalimetallen, bei dem metallisches Calcium, Strontium, Barium oder deren Hydride oder Acetylide mit einem niederen Alkanol mit 1 bis 7 Kohlenstoffatomen zu einem Metallalkoxid umgesetzt werden, dadurch gekennzeichnet, daß zu dem erhaltenen Reaktionsprodukt ein Polyol oder ein höherer aliphatischer, cycloaliphatischer oder phenylsubstituierter aliphatischer einwertiger Alkohol mit mindestens 4 Kohlenstoffatomen, der weniger flüchtig ist als das niedere Alkanol, gegeben und das Alkanol aus dem Gemisch entfernt wird.

2. Verfahren nach Anspruch 1, worin die Menge des Polyols oder weniger flüchtigen aliphatischen, cycloaliphatischen oder phenyl-substituierten aliphatischen einwertigen Alkohols, die zu dem $C_{1-7}$-Alkanol-Erdalkalimetallalkoxid-Reaktionsprodukt gegeben wird, eine ausreichende Menge ist, um das gesamte $C_{1-7}$-Alkanol-Metallalkoxid umzuwandeln.

3. Verfahren nach Anspruch 1, worin die Erdalkalimetallkonzentration des $C_{1-7}$-Alkanol-Reaktionsgemisches 0,01 bis 20 Gewichtsprozent beträgt.

4. Verfahren nach Anspruch 1, worin ein Polyol mit dem $C_{1-7}$-Alkanol-Erdalkalimetallalkoxid-Reaktionsgemisch vermischt wird.

5. Verfahren nach Anspruch 4, worin das Polyol 2 bis 30 Kohlenstoffatome und 2 bis 6 Hydroxylgruppen aufweist.

6. Verfahren nach Anspruch 1, worin ein weniger flüchtiger aliphatischer, cycloaliphatischer oder phenyl-substituierter aliphatischer einwertiger Alkohol mit dem $C_{1-7}$-Alkanol-Erdalkalimetallalkoxid-Reaktionsgemisch vermischt wird.

7. Verfahren nach Anspruch 6, worin der weniger flüchtige aliphatische, cycloaliphatische oder phenyl-substituierte aliphatische einwertige Alkohol zwischen 8 und 30 Kohlenstoffatome enthält.

**Revendications**

1. Procédé de préparation de composés catalytiquement actifs de métaux alcalino-terreux, dans lequel du calcium, du strontium, du baryum métalliques ou leurs hydrures ou acétylures sont amenés à réagir avec un alcanol inférieur ayant 1 à 7 atomes de carbone, pour former un alcoolate métallique, caractérisé en ce qu'on ajoute au produit réactionnel ainsi obtenu un polyol ou un alcool monohydroxylique aliphatique supérieur, cycloaliphatique ou aliphatique à substituant phényle, qui a au moins 4 atomes de carbone et qui est moins volatil que l'alcanol inférieur, et en ce qu'on élimine l'alcanol du mélange.

2. Procédé suivant la revendication 1, dans lequel la quantité de polyol ou d'alcool monohydroxylique aliphatique, cycloaliphatique ou aliphatique à substituant phényle moins volatil ajouté à l'alcoolate de métal alcalino-terreux d'alcanol en $C_1$ à $C_7$ obtenu comme produit de réaction est une quantité suffisante pour convertir la totalité de l'alcoolate métallique d'alcanol en $C_1$ à $C_7$.

3. Procédé suivant la revendication 1, dans lequel la concentration du métal alcalino-terreux dans le mélange réactionnel de l'alcanol en $C_1$ à $C_7$ va de 0,01 à 20% en poids.

4. Procédé suivant la revendication 1, dans lequel un polyol est mélangé avec le mélange réactionnel formé de l'alcoolate de métal alcalino-terreux et de l'alcanol en $C_1$ à $C_7$.

5. Procédé suivant la revendication 4, dans lequel le polyol comprend 2 à 30 atomes de carbone et 2 à 6 groupes hydroxyle.

6. Procédé suivant la revendication 1, dans lequel un alcool monohydroxylique aliphatique, cycloaliphatique ou aliphatique à substituant phényle moins volatil est mélangé avec le mélange réactionnel formé de l'alcoolate de métal alcalino-terreux et de l'alcanol en $C_1$ à $C_7$.

7. Procédé suivant la revendication 6, dans lequel l'alcool monohydroxylique aliphatique, cycloaliphatique ou aliphatique à substituant phényle moins volatil comprend entre 8 et 30 atomes de carbone.